# EUROPEAN PATENT SPECIFICATION

(11) **EP 0 540 747 B1**
(45) Date of publication and mention of the grant of the patent: **09.09.1998**
(21) Application number: 92908283.2
(22) Date of filing: 20.04.1992
(51) Int. Cl.: A61K 45/00, A61K 38/46, A61K 9/08, A61K 47/36

(54) **MEDICINE FOR INTRAOCULAR OPERATION**
MEDIZIN ZUR INTRAOKULAREN OPERATION
MEDICAMENT UTILISE EN CHIRURGIE INTRA-OCULAIRE

(30) Priority: 22.04.1991 JP 185948/91
(43) Date of publication of application: 12.05.1993
(73) Proprietor: Senju Pharmaceutical Co., Ltd., Osaka-shi, Osaka 541 (JP)
(72) Inventor: SHIMIZU, Hiroyuki, 1-3-15, Koun-cho, Gunma 371 (JP); OGAWA, Takahiro, 8-23, Atagoyama, Hyogo 662 (JP)
(74) Representative: Burford, Anthony Frederick
(86) International application number: JP9200509
(87) International publication number: WO9218163

(56) References cited:
- JP-A- 2 036 128
- JP-A- 2 164 828
- JP-A- 2 167 233
- ACTA MEDICA ET BIOLOGICA vol. 15, no. 1, 1967, JAPAN pages 31 - 41 M. MIKUNI ET AL. 'OPHTHALMIC USE OF UROKINASE'
- DATABASE WPIL Week 9206, Derwent Publications Ltd., London, GB; AN 92-045976 & JP-A-3 291 240 (SANTEN PHARM KK ET AL.) 20 December 1991
- DATABASE WPI Week 7928, Derwent Publications Ltd., London, GB; AN 79-51401B & JP-A-54 067 089 (DAINIPPON PHARM KK) 30 May 1979
- CHEMICAL ABSTRACTS, vol. 91, 1979, Columbus, Ohio, US; abstract no. 86500b, MORI TOSHIO 'STABLE UROKINASE SOLUTION' page 354 ;column 1 ;
- CHEMICAL ABSTRACTS, vol. 77, no. 10, 4 September 1972, Columbus, Ohio, US; abstract no. 69862q, M.G. MARGOLIS 'ENZYMES AND COENZYMES IN OPHTHALMOLOGY' page 1 ;column 1 ;
- EXPERIMENTAL EYE RESEARCH vol. 11, no. 2, 1971, pages 178 - 183 H. SAIDUZZAFAR ET AL. 'THE EFFECT OF INTRACAMERAL AND INTRAVENOUS UROKINASE ON THE RESISTANCE TO AQUEOUS OUTFLOW IN CYNOMOLOGUS MONKEYS'
- AM. J. OPHTHALMOL. vol. 84, no. 1, 1977, pages 79 - 84 D.M. LEET 'TREATMENT OF TOTAL HYPHEMAS WITH UROKINASE'
- P.H. LIST 'ARZNEIFORMENLEHRE' 1982 , WISSENSCHAFTL. VERLAGSGES. MBH , STUTTGART
- CHEMICAL ABSTRACTS, Vol. 114, No. 5, 4 February 1991 (04.02.91), PAUL STERNBERG, Jr. et al. "Refer to the effect of tissue plasminogen activator on retinal-bleeding", page 39, Abstract No. 35632g, Arch. Ophtalmol, (Chicago), 1990, 108(5), pages 720-722.

## Description

The present invention relates to the use of urokinase, preferably with a biocompatible viscoelastic compound of high molecular weight, to effectively inhibit ocular hypertension following intraocular surgery such as an operation for intraocular lens implantation.

To complete cataract therapy, implantation of a pseudophakos or intraocular lens is performed. However, even the slightest contact of the intraocular lens with the corneal surface while placing the lens may induce corneal endothelial disorder, corneal edema and even aphakic bullous keratophathy and, therefore, it is common practice to administer an auxiliary agent for preoperative management at IOL surgery for prevention of such complications. Such auxiliary agents generally contain a biocompatible polymer such as hyaluronic acid or a salt thereof, chondroitinsulfuric acid or a salt thereof, or methylcellulose.

However, after completion of an operation for IOL implantation with the aid of such an auxiliary agent, the auxiliary agent must be washed out from the eye by irrigating the anterior chamber. If this irrigation is insufficient, the auxiliary agent will remain in the eye to elicit abnormal elevation of intraocular pressure, corneal opacity, and various disorders associated with compressed optic nerves, with blindness resulting in extreme cases. It is, therefore, an essential procedure to inhibit ocular hypertension following IOL implantation surgery and, for this purpose, a drug for inhibiting postoperative elevation of intraocular pressure, such as timolol or acetazolamide, conventionally is administered.

However, reports on these drugs are not unanimous as to their efficacy in the prevention and therapy of ocular hypertension following intraocular surgery, such as an operation for IOL implantation, and their effectiveness is still in doubt, with no surgeon being fully certain of their antihypertensive effect. It is against this background that the advent of a drug capable of preventing such elevation of intraocular pressure following intraocular surgery, such as an operation for IOL implantation, has been earnestly awaited.

Under the circumstances, the Inventors of the present invention, who have been energetically seeking a drug that will effectively inhibit postoperative ocular hypertension following ophthalmic surgery, such as an IOL operation, without exerting untoward effects on the eye. As a result, they discovered that urokinase is effective in inhibiting postoperative elevation of intraocular pressure and have developed the present invention.

JP-A-54067089 discloses that salts of chondroitinsulfuric acid stabilize solutions of urokinase. The compositions are stated to be suitable for injection or administration as eye-drops. It states that urokinase activates plasminogen in serum to convert it into plasmin having fibrin-dissolving power and is useful as an antithrombotic agent.

JP-A-7955782 discloses the use of sulfate sugar esters to stabilize urokinase solutions. Specified esters include chondroitin sulfate.

Mikuni et al (Acta Med. Biol. 15(1), 1967, 31-41; D3) discusses ophthalmic uses of urokinase. These uses include continuous intravenous infusion to treat preretinal haemorrhage and retinal artery or vein obstruction and local application to treat various haemorrhagic eye diseases. Local treatment with urokinase was found to provide very satisfactory improvement in three (of three) patients with spontaneous subconjunctival haemorrhages and two (of two) patients with traumatic subconjunctival haemorrhages and a favourable effect in one (of one) patient with postoperative vitreous haemorrhage but little or no effect in three (of three) patients suffering arteriosclerosis vitreous haemorrhages and two (of two) patients with haemorrhages at macula due to hypertension. Four (of fourteen) patients with retinal vein haemorrhages were treated effectively by local administration but the remaining ten patients showed only slightly effective results. There is on reference to any stabilization of the urokinase solutions used.

It was not hitherto known that urokinase inhibits ocular hypertension following an operation for intraocular lens implantation. Accordingly, the invention provides the use of urokinase in the manufacture of a composition for inhibiting postoperative ocular hypertension.

In a preferred embodiment, the urokinase is used as an intraocular auxiliary composition comprising urokinase and a biocompatible viscoelastic compound of high molecular weight such as hyaluronic acid and its salts, chondroitinsulfuric acid and its salts, and methylcellulose.

The invention also provides an intraocular auxiliary composition comprising urokinase and methylcellulose.

The urokinase is preferably used dissolved in an aqueous medium. The said aqueous medium may be any medium that does not adversely affect the ocular tissues on topical application to the eye. Thus, it may, for example, be purified water, physiological saline, or an appropriate buffer solution. As said buffer solution, phosphate buffer or acetate buffer advantageously can be employed. Urokinase is dissolved in such an aqueous medium and the solution adjusted to a pH value close to that of the aqueous humor of the eye. In other words, the composition is preferably constituted to have a pH value between 6 and 7.5.

Known species of urokinase have molecular weights in the neighborhood of 53,000 and 33,000, respectively, and either or both of them can be employed in accordance with the present invention. In performing IOL surgery, urokinase is injected via the cornea into the anterior chamber. The injection dose can be selected according to, inter alia, the particular species of urokinase and the formulation of the particular composition. It is preferably injected into the anterior chamber in a dose of generally 50 to 300 international units (I.U.) and preferably 100 to 200 I.U. In addition to urokinase, the composition optionally contains a biocompatible viscoelastic compound of high molecular weight. The term 'biocompatible' means that the particular substance can be injected or otherwise administered into a living body without causing untoward effects on the various tissues of the body and, in a preferred sense, refers to cases in which the substance is metabolized or absorbed and thereafter exerts no serious adverse effect on the body. Of course, those compounds of high molecular weight which are readily excreted from the body after injection are also biocompatible in the context of the present invention. Thus, any appropriate compound of high molecular weight that is viscoelastic and biocompatible can be employed for purposes of the invention. For example, hyaluronic acid and its salts, chondroitinsulfuric acid and its salts, and methylcellulose can all be employed with advantage. While these biocompatible viscoelastic compounds of high molecular weight have for some time been employed as surgical auxiliary agents in ophthalmic operations such as intraocular lens implantation, of course any other compounds can likewise be employed if they do not adversely affect the ocular tissues after injection into the eye.

Hyaluronic acid and chondroitinsulfuric acid can each be used in the free form or as a salt. The salt may be, for example, the corresponding salt of an alkali metal, e.g. sodium and potassium, or of an alkaline earth metal, e.g. calcium or barium.

Hyaluronic acid species having molecular weights in the range of 300,000 to 4,000,000 can be harvested from animal tissues or microbial cultures and any of these species can be used as an ingredient of the composition. Chondroitinsulfuric acid is available in the molecular weight range of 10,000 to 50,000 and any of such species can be successfully employed. Methylcellulose is available in the molecular weight range of 9,000 to 270,000 and any of such species can be likewise employed.

While the concentration of such a biocompatible high molecular compound is dependent on, inter alia, its molecular weight and the clinical condition of the patient to be treated, the generally preferred concentration is 0.1 to 10% for hyaluronic acid, about 20% for chondroitinsulfuric acid, or about 2% for methylcellulose. These biocompatible compounds of high molecular weight can be used independently or in suitable combinations unless the object of the invention is frustrated.

In using the composition in an operation for intraocular lens implantation, it is preferably injected through the limbal incision of the cornea using an injection syringe having an appropriate caliber. Therefore, the composition is preferably prepared with a viscosity which permits injection with a syringe into the anterior chamber.

Depending on the predicted degree of postoperative ocular hypertension in each individual case and other conditions, the composition can be used in combination with other intraocular pressure modulating agents. Furthermore, unless the object of the invention is frustrated, any drugs that are used in routine ophthalmological practice, such as antiinflammatory agents and antimicrobial agents, can be incorporated in this composition.

Because the composition reliably inhibits postoperative ocular hypertension with great certainty when injected into the eye in ophthalmic surgery such as an operation for IOL implantation, it can be used with great advantage especially in an operation for intraocular lens implantation. Moreover, when a biocompatible viscoelastic compound of high molecular weight is incorporated in this composition, there is made available an auxiliary composition for IOL implantation which effectively prevents postoperative ocular hypertension.

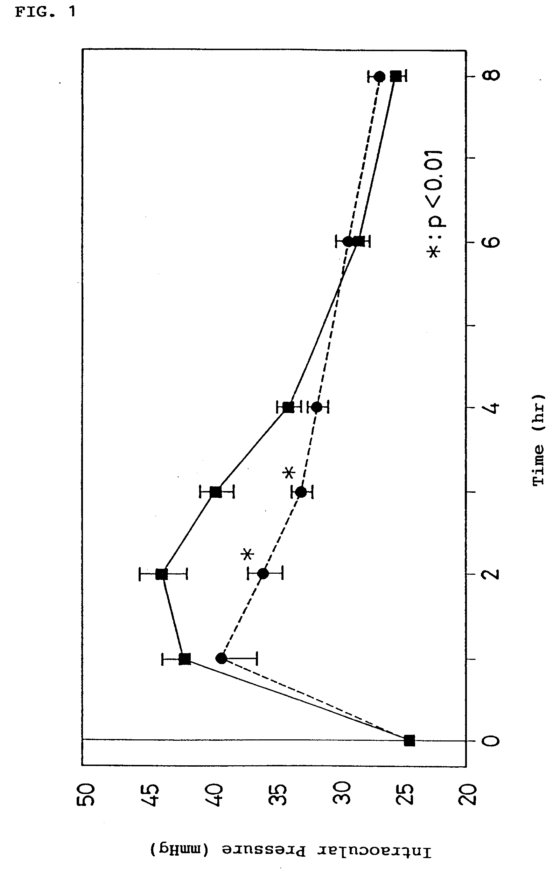

Fig. 1 shows the change over time in the intraocular pressure of rabbits in Experimental Example 1, with the intraocular pressure (mmHg) (kPa) being plotted on the vertical axis against the time (hours) on the horizontal axis.
● ...● represents the intraocular pressure of the eyes injected with a physiological saline solution containing both urokinase and sodium hyaluronate.
■ ...■ represents the intraocular pressure of the control eyes injected with a physiological saline solution containing hyaluronic acid alone.

The following experimental example and examples are further illustrative of the present invention.

### Experiment 1

### Effect of urokinase on the elevation of intraocular pressure following an injection of sodium hyaluronate into the rabbit anterior chamber

### Method

- Animal:: Albino rabbits were used.
- Drug :: 1% sodium hyaluronate (molecular weight 2,000,000)

The urokinase was a reagent-grade preparation with a potency of 3000 I.U. available from Green Cross Corporation (mannitol contained as the excipient).
Experiment: Using albino rabbits (2.2-3.0 kg), 150 µl of aqueous humor was collected from the anterior chamber by corneal paracentesis with a 30G needle, followed immediately by injection of 150 µl of physiological saline containing sodium hyaluronate. The fellow eye was similarly treated with 150 µl of a urokinase-containing sodium hyaluronate solution (one volume of 100 I.U. of urokinase was added to 9 volumes of sodium hyaluronate). At 1, 2, 3, 4, 6 and 8 hours after injection, the intraocular pressure was measured with a PTG (Pneumatonograph, Alcon).

The results are shown in Fig. 1. It is apparent from Table 1 that urokinase effectively inhibits ocular hypertension following injection of sodium hyaluronate into the anterior chamber.

### Example 1

100 I.U. of urokinase having a molecular weight of 53,000 is dissolved in 0.1 ml of physiological saline and the solution is used as an auxiliary composition in an operation for intraocular lens implantation.

### Example 2

4 mg of sodium hyaluronate having a molecular weight of 700,000--4,000,000 and 100 I.U. of an urokinase having a molecular weight of 53,000 are dissolved in 0.4 ml of physiological saline and the solution is used as an auxiliary composition in an operation for intraocular lens implantation.

### Example 3

80 mg or sodium chondroitinsulfate having a molecular weight of 75,000-500,000 and 100 I.U. of an urokinase having a molecular weight of 53,000 are dissolved in 0.4 ml of an aqueous buffer solution and the solution is used as an auxiliary composition in an operation for intraocular lens implantation.

### Example 4

4 mg of a methylcellulose having a molecular weight of 20,000 to 500,000 and 100 I.U. of an urokinase having a molecular weight of 53,000 are dissolved in 0.4 ml of physiological saline and the solution is used as an intraocular surgery auxiliary composition in an operation for intraocular lens implantation.

## Claims

1. The use of urokinase in the manufacture of a composition for inhibiting postoperative ocular hypertension.

2. A use as claimed in Claim 1, wherein said composition further comprises a biocompatible viscoelastic compound of high molecular weight.

3. A use as claimed in Claim 2, wherein said biocompatible viscoelastic compound is at least one member selected from hyaluronic acid and its salts, chondroitinsulfuric acid and its salts, and methylcellulose.

4. An intraocular auxiliary composition comprising urokinase and methylcellulose.

5. An intraocular auxiliary composition as claimed in Claim 4 comprising an aqueous vehicle.

6. An intraocular auxiliary composition as claimed in Claim 5, wherein the pH of the composition is between pH 6 and pH 7.5

7. An intraocular auxiliary composition as claimed in Claim 5 or Claim 6 having a viscosity permitting of injection by syringe into the anterior chamber of the eye.

8. A use as claimed in Claim 3, wherein said composition comprises an aqueous vehicle.

9. A use as claimed in Claim 8, wherein the pH of the composition is between pH 6 and pH 7.5

10. A use as claimed in Claim 8 or Claim 9, wherein said composition has a viscosity permitting of injection by syringe into the anterior chamber of the eye.

## Patentansprüche

1. Verwendung von Urokinase bei der Herstellung einer Zusammensetzung zum Verhindern eines postoperativen Augenhochdrucks.

2. Verwendung nach Anspruch 1, wobei die Zusammensetzung weiterhin eine biologisch kompatible, viskoelastische Verbindung von hohem Molekulargewicht aufweist.

3. Verwendung nach Anspruch 2, wobei die biologisch kompatible, viskoelastische Verbindung mindestens eines der folgenden Elemente ist: Hyaluronsäure und deren Salze, Chondroitin-Schwefelsäure und deren Salze, und Methylcellulose.

4. Intraokulare Hilfs-Zusammensetzung, welche Urokinase und Methylcellulose aufweist.

5. Intraokulare Hilfs-Zusammensetzung nach Anspruch 4, welche einen wässrigen Träger aufweist.

6. Intraokulare Hilfs-Zusammensetzung nach Anspruch 5, wobei der pH-Wert der Zusammensetzung zwischen pH 6 und pH 7,5 liegt.

7. Intraokulare Hilfs-Zusammensetzung nach Anspruch 5 oder 6, welche eine solche Viskosität hat, daß das Einspritzen mittels einer Spritze in die vordere Augenkammer möglich ist.

8. Verwendung nach Anspruch 3, wobei die Zusammensetzung einen wässrigen Träger aufweist.

9. Verwendung nach Anspruch 8, wobei der pH-Wert der Zusammensetzung zwischen pH 6 und pH 7,5 liegt.

10. Verwendung nach Anspruch 8 oder Anspruch 9, wobei die Zusammensetzung eine solche Viskosität hat, daß das Einspritzen mittels einer Spritze in die vordere Augenkammer möglich ist.

## Revendications

1. Utilisation d'urokinase dans la fabrication d'une composition pour inhiber l'hypertension oculaire postopératoire.

2. Utilisation selon la revendication 1, dans laquelle ladite composition comprend en outre un composé viscoélastique biocompatible de haut poids moléculaire.

3. Utilisation selon la revendication 2, dans laquelle ledit composé viscoélastique biocompatible est au moins un élément choisi parmi l'acide hyaluronique et ses sels, l'acide chondroitinesulfurique et ses sels, et la méthylcellulose.

4. Composition intra-oculaire auxiliaire comprenant de l'urokinase et de la méthylcellulose.

5. Composition intra-oculaire auxiliaire selon la revendication 4, comprenant un véhicule aqueux.

6. Composition intra-oculaire auxiliaire selon la revendication 5, dans laquelle le pH de la composition est entre pH 6 et pH 7,5.

7. Composition intra-oculaire auxiliaire selon la revendication 5 ou la revendication 6, ayant une viscosité permettant de l'injecter par une seringue dans la chambre antérieure de l'oeil.

8. Utilisation selon la revendication 3, dans laquelle ladite composition comprend un véhicule aqueux.

9. Utilisation selon la revendication 8, dans laquelle le pH de la composition est entre pH 6 et pH 7,5.

10. Utilisation selon la revendication 8 ou la revendication 9, dans laquelle ladite composition a une viscosité permettant de l'injecter par une seringue dans la chambre antérieure de l'oeil.
